(19)

Europäisches
Patentamt
European
Patent Office
Office européen
des brevets

(11) **EP 4 512 824 A1**

(12) **EUROPEAN PATENT APPLICATION**
published in accordance with Art. 153(4) EPC

(43) Date of publication:
**26.02.2025 Bulletin 2025/09**

(21) Application number: **23841813.1**

(22) Date of filing: **10.04.2023**

(51) International Patent Classification (IPC):
*C07K 14/78* (2006.01)  *C07K 1/18* (2006.01)
*C12N 15/12* (2006.01)  *C12N 1/21* (2006.01)
*C12N 15/63* (2006.01)  *C12N 15/70* (2006.01)
*A61K 8/65* (2006.01)  *A61K 38/39* (2006.01)
*A61L 31/04* (2006.01)  *A61K 47/42* (2017.01)
*A61L 27/24* (2006.01)  *A23L 33/17* (2016.01)
*A61L 15/32* (2006.01)  *A61Q 19/00* (2006.01)
*C07K 19/00* (2006.01)

(86) International application number:
**PCT/CN2023/087212**

(87) International publication number:
**WO 2024/016740 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.07.2022 CN 202210849498**

(71) Applicant: **Shanxi Jinbo Bio-Pharmaceutical Co.,
Ltd.
Shanxi 030032 (CN)**

(72) Inventors:
• **YANG, Xia
Taiyuan, Shanxi 030032 (CN)**

• **ZHANG, Xingdong
Taiyuan, Shanxi 030032 (CN)**
• **WANG, Yunbing
Taiyuan, Shanxi 030032 (CN)**
• **YANG, Li
Taiyuan, Shanxi 030032 (CN)**
• **HE, Zhenrui
Taiyuan, Shanxi 030032 (CN)**

(74) Representative: **Cabinet Becker et Associés
25, rue Louis le Grand
75002 Paris (FR)**

Remarks:
The complete document including Reference
Table(s) and the Sequence Listing(s) can be
downloaded from the EPO website

(54) **BIOSYNTHESIS-BASED PREPARATION METHOD FOR STRUCTURAL MATERIAL OF HUMAN
BODY**

(57) Provided is a biosynthesis-based preparation method for a structural material of the human body. A polypeptide of the present application contains the structure of a C-terminal region of (repeating unit)n or (repeating unit)n-; the repeating unit contains an amino acid sequence as shown in SEQ ID NO. 1. Recombinant type V humanized collagen prepared in the present application has high activity in promoting cell adhesion, and does not cause an immune response when applied to the human body; moreover, the preparation method is novel, allows for large-scale production of the recombinant type V humanized collagen, and is widely used in the preparation of structural materials of the human body. The application field includes preparation of high-end medical devices, such as biological dressings, human body biomimetic materials, plastic-beauty materials, organoid culture, cardiovascular stents, coatings, tissue injection filling, ophthalmic materials, gynecological biomaterials, nerve repair and regeneration, liver tissue and vessel repair and regeneration, and 3D printing of artificial organ biomaterials.

EP 4 512 824 A1

Fig. 4

## Description

The present application claims the priority to Chinese Invention Patent Application No. 202210849498.3 titled "BIOSYNTHESIS-BASED PREPARATION METHOD FOR STRUCTURAL MATERIAL OF HUMAN BODY", filed on July 19, 2022, which is incorporated herein by reference.

## Technical Field

The present application belongs to the field of synthetic biotechnology and relates to a biosynthesis-based preparation method for structural material of human body.

## Background

The structural materials of the human body are mainly structural proteins including collagen. Such proteins have adhesion and support functions for cells and tissues and are the main component of the extracellular matrix. Collagen is a type of protein widely distributed in human connective tissues and is also the most abundant protein in the human body, accounting for 25% to 35% of the total protein. It is currently found that there are at least 28 collagen subtypes in the human body, which are located in different tissues or organs. Among them, type V collagen belongs to fibrous collagen, which is often expressed along with type I collagen, with relatively low content. Type V collagen possesses unique physiological functions, such as inhibiting the adhesion and proliferation of epidermis, endothelium, smooth muscle and cancer cells, and binding with heparin, insulin, osteonectin, vascular endothelial inhibitory factor, macrophage colony-stimulating factor and other biologically active substances, which can be used in the fields of medicine, materials, and biomedicine.

In recent years, crude type V collagen has been extracted mainly from a variety of aquatic products or tissues such as placenta and kidney tissues. Unfortunately, since type V collagen has a relatively low content in an organism and belongs to a secondary collagen, the extraction process of type V collagen is relatively complicated. At the same time, animal-derived immune responses are also an important reason for the limitation of collagen in application. With the increasing growth of the collagen industry in our country, the utilization of biosynthetic pathways to obtain collagen has become increasingly mature, especially in humanized collagen, which has been at the forefront of the world. In 2021, the National Medical Products Administration conducted the naming and classification of biosynthetic collagen. Among them, recombinant humanized collagen refers to the full-length or partial amino acid sequence fragment encoded by the gene of the specific type of human collagen prepared by DNA recombinant technology, or a combination containing function fragments of human collagen.

Type V collagen was first found in the human placenta and skin. It contains three $\alpha$ chains of different strengths and mobility, which can form four different subtypes in tissues, namely $\alpha1\alpha1\alpha2(V)$, $\alpha1\alpha2\alpha3(V)$, $\alpha3\alpha3\alpha3(V)$, and a mixture of type V collagen $\alpha$ chain and type XI collagen $\alpha$ chain. Three different $\alpha$ chains are combined to form triple strands of rope-like procollagen molecules, which are assembled into a slender collagen fiber after the treatment of extracellular enzyme digestion. The slender fibers are cross-linked with each other in the space around the cells, forming a high-strength mature type V collagen fiber network after crosslinking; alternatively, they are combined with other types of collagen such as type XI collagen to form a heterotrimer, which is distributed in the cornea, skin, ligament, bone, tendon, muscle and other sites. Type V collagen has the common structural characteristics of fibrillar collagen, with an uninterrupted sequence of more than 1,000 glycine-Xaa-Yaa in the central region, wherein the Xaa-Yaa is any amino acid or imino acid residue. Structurally, the structure of natural type V collagen in the human body is very complex, which makes it difficult to express and prepare the humanized collagen on a large scale through conventional means.

Currently, type V collagen has been mainly produced by processing animal-derived tissues with pepsin to extract collagen derivatives. However, the collagen extracted by these methods has its original biological activity, and cannot play the true function of such collagen. With the development of modern biotechnology, one has been able to prepare recombinant humanized collagen in animal, plant, and microbial expression systems through transgenic technology, solving the disadvantages of the enzymatic method. However, at present, recombinant type V humanized collagen has not been successfully obtained. Therefore, there is an urgent need for a biosynthetic method for recombinant type V humanized collagen so that it can be widely applied as a structural material of the human body.

## Summary of the Invention

In view of the technical problems presented in the prior art, the inventors have designed for the first time a functional region screening and protein synthesis process for recombinant type V humanized collagen. The inventors have found the amino acid sequence of the core region of type V collagen (SEQ ID NO. 1, gkegtkgdpgpaglpgkdgppglrgfpgdrglpgpv), and constructed a polypeptide comprising a plurality of repeating units with this core region as a repeating unit. The inventors have discovered that the constructed polypeptide has the activity of promoting cell adhesion. The inventors have also found that one or more amino acids may be extended at the N-terminus and/or C-terminus of the core region as a repeating unit, and the constructed polypeptide comprising a plurality of repeating units also possesses the activity of promoting cell adhesion. Further, in addition to the repeating units, the polypeptide may comprise a peptide segment of a certain length outside the repeating units (for example, the N-terminus and/or C-terminus of the polypeptide composed of the repeating units). The peptide segment may be a consecutive amino acid segment starting from position 1 of the repeating unit.

In one aspect, the present application provides a polypeptide comprising a structure of (repeating unit)$_n$ or (repeating unit)$_n$ -C-terminal region, wherein the repeating unit comprises either the amino acid sequence of SEQ ID NO. 1, or SEQ ID NO. 1 and an additional amino acid residue at the N-terminus and/or C-terminus of SEQ ID NO. 1, and the number of the additional amino acid residue is 1-50. The polypeptide of the present application is recombinant type V humanized collagen.

In one embodiment, each repeating unit is directly linked and the number n of the repeating unit is 4-20, and the C-terminal region is a consecutive amino acid segment starting from position 1 of the repeating unit.

In one embodiment, the additional amino acid residue is at the C-terminus of SEQ ID NO. 1 and is the amino acid sequence set forth in SEQ ID NO. 7 (galglk) or a consecutive amino acid segment thereof. For example, the additional amino acid residue is a consecutive amino acid segment starting from position 1 of the amino acid sequence set forth in SEQ ID NO. 7.

In one embodiment, the repeating unit comprises the amino acid sequence of any one of SEQ ID NOs. 2, 3 and 8-11.

In one embodiment, the polypeptide comprises an amino acid sequence selected from SEQ ID NOs. 4-6.

In another aspect, the present application provides a nucleic acid comprising nucleotides of the polypeptide of the present application.

In one embodiment, the nucleic acid further comprises nucleotides encoding a purification tag, e.g. a His tag, a GST tag, an MBP tag, a SUMO tag, or a NusA tag.

In one embodiment, the nucleic acid further comprises nucleotides encoding a leader sequence.

In another aspect, the present application provides a vector comprising the nucleic acid according to the present application.

In one embodiment, the vector comprises an expression control element, such as a promoter, a terminator, and/or an enhancer, operably linked to the nucleic acid.

In another aspect, the present application provides a host cell comprising the nucleic acid or the vector of the present application. In one embodiment, the host cell is a eukaryotic or a prokaryotic cell. In one embodiment, the eukaryotic cell is a yeast cell, an animal cell, and/or an insect cell. In one embodiment, the prokaryotic cell is an *Escherichia coli* cell.

In another aspect, the present application provides a methosd for producing the polypeptide of the present application, comprising:

> (1) culturing the host cell of the present application under a suitable culture condition;
> (2) harvesting the host cells and/or culture medium comprising the polypeptide; and
> (3) purifying the fusion protein or trimeric fusion protein.

In another aspect, the present application provides compositions comprising the polypeptide, the nucleic acid, the vector and/or the host cell of the present application.

In one embodiment, the composition is one or more of biological dressings, human body bionic materials, plastic beauty materials, organoid culture materials, cardiovascular stent materials, coating materials, tissue injection filling materials, ophthalmic materials, obstetrics and gynecology biomaterials, nerve repair and regeneration materials, liver tissue materials and vascular repair and regeneration materials, 3D printed artificial organ biomaterials, cosmetic raw materials, pharmaceutical excipients and food additives.

In another aspect, the present application provides use of the polypeptide, the nucleic acid, the vector, the host cell and/or the composition of the present application for use in promoting cell adhesion *in vitro* or in the manufacture of a medicament for promoting cell adhesion.

In another aspect, the present application provides application of the polypeptide, the nucleic acid, the vector, the host cell and/or the composition of the present application for the manufacture of high-end medical devices, such as biological dressings, human body bionic materials, plastic beauty materials, organoid culture, cardiovascular stent, coating, tissue injection filling, ophthalmic materials, obstetrics and gynecology biomaterials, nerve repair and regeneration, liver tissue and vascular repair and regeneration, 3D printed artificial organ biomaterials; high-end cosmetic raw materials and high-end pharmaceutical excipients; and food additives.

The present application discloses a specific process of functional region screening and synthesis process of a recombinant type V humanized collagen, which may be used for the preparation of structural materials of the human body.

The present application belongs to the field of synthetic biology technology. The present application discloses a specific process of functional region screening and synthesis process of a recombinant type V humanized collagen, which may be used for the preparation of structural materials of the human body. The present application belongs to the field of synthetic biology technology. The biosynthetic method for preparing the recombinant type V humanized collagen in the present application comprises the specific processes as follows: (1) functional region screening and strain construction; (2) biological fermentation, induction and expression; (3) purification and optional enzyme digestion of the humanized type V collagen. The amino acid sequence of the recombinant humanized collagen prepared in the present application is derived from a functional region of human natural type V collagen, and comprises the functional region and a similar functional region, as well as a protein with the amino acid sequence mutated and modified respectively. The recombinant type V humanized collagen prepared in the present application has high activity in promoting cell adhesion, and does not cause an immune response when applied to the human body. The preparation method of the recombinant type V humanized collagen is novel. The recombinant type V humanized collagen may be obtained on a large scale and is widely used in the preparation of human body structural materials. Its application field includes the preparation of high-end medical devices, such as biological dressings, human body bionic materials, plastic beauty materials, organoid culture, cardiovascular stent, coating, tissue injection filling, ophthalmic materials, obstetrics and gynecology biomaterials, nerve repair and regeneration, liver tissue and vascular repair and regeneration, 3D printed artificial organ biomaterials; high-end cosmetic raw materials and high-end pharmaceutical excipients; food additives and the like.

The present application provides the following:

1. In view of the current research status, the present application provides a method for biosynthesizing recombinant type V humanized collagen, that is, a method for preparing structural materials of human body. The specific processes comprises: (1) functional region screening and strain construction; (2) large-scale biological fermentation culture and inducible expression of the protein; and (3) purification and optional enzyme digestion of the humanized type V collagen.

2. According to item 1, functional region screening and strain construction may be conducted as follows: (1) screening functional regions on a large scale to obtain a functional region of the target gene; (2) inserting the obtained functional region of the target gene into a pET-32a expression vector to obtain a recombinant expression plasmid; and (3) transforming the recombinant expression plasmid into *Escherichia coli* competent cells BL21 (DE3) to obtain positive *Escherichia coli* genetically engineered bacteria by screening.

3. According to item 1, large-scale biological fermentation may be conducted as follows: adding the positive *E. coli* genetically engineered bacteria obtained by screening to a shake flask containing an antibiotic stock solution, and culturing the bacteria in a constant temperature shaker at 220 rpm and 37°C for 7h.

4. According to item 1, the inducible expression of the protein may be conducted as follows: (1) cooling the shake flask after the culture to 16°C; (2) adding IPTG stock solution for inducible expression; and (3) placing the bacterial liquid after inducible expression into a centrifuge bottle, and collecting the bacterial cells after centrifugation at 8000 rpm and 4°C for 10 min.

5. According to item 1, the purification and optional enzyme digestion of the humanized type V collagen may be conducted as follows: (1) crudely purifying the humanized type V collagen on a Ni affinity chromatography column; (2) adding TEV enzyme at a certain ratio for enzyme digestion; and (3) finely purifying the humanized type V collagen on an ion exchange column.

6. According to item 2, the screened functional regions are set forth as follows: (1) C5V3G1 amino acid sequence: gkegtkgdpgpaglpgkdgppglrgfpgdrglpgpvgalglkgkegtkgdpgpaglpgkdgppglrgfpgdr glpgpvgalglkgkegtkgdpgpaglpgk dgppglrgfpgdrglpgpvgalglkgkegtkgdpgpaglpgkd gppglrgfpgdrglpgpvgalglkgkegtkgdpgpaglpgkdgppglrgfpgdrglpgp vgalglkgkegtkg dpgpaglpgkdgppglrgfpgdrglpgpvgalglkgkegtkgdpgpaglpgkdgppglrgfpgdrglpgpv alglkgkegtkgdpg- paglpgkdgppglrgfpgdrglpgpvgalglk; (2) C5V3G2 amino acid sequence: gkegtkgdpgpaglpgkdgppglrgfpgdrglpgpvg algkegtkgdpgpaglpgkdgppglrgfpgdrglp gpvgalgkegtkgdpgpaglpgkdgppglrgfpgdrglpgpvgalgkegtkgdpgpaglpgkdgp pglrgfp gdrglpgpvgalgkegtkgdpgpaglpgkdgppglrgfpgdrglpgpvgalgkegtkgdpgpaglpgkdgp pglrgfpgdrglpgpvgalgkegt kgdpgpaglpgkdgppglrgfpgdrglpgpvgalgkegtkgdpgpagl pgkdgppglrgfpgdrglpgpvgal; and (3) C5V3G3 amino acid sequence:

gkegtkgdpgpaglpgkdgppglrgfpgdrglpgpvgkegtkgdpgpaglpgkdgppglrgfpgdrglpgp

vgkegtkgdpgpaglpgkdgppglrgfpgdrglpgpvgkegtkgdpgpaglpgkdgppglrgfpgdrglpg

pvgkegtkgdpgpaglpgkdgppglrgfpgdrglpgpvgkegtkgdpgpaglpgkdgppglrgfpgdrglp

gpvgkegtkgdpgpaglpgkdgppglrgfpgdrglpgpvgkegtkgdpgpaglpgkdgppglrgfpgdrgl

pgpvgkegtkgdpgpaglpgkdgppglrgfpgdrglpgpvgkegtkgdpgpaglpgkdgppglrgfpgdrg

lpgpv.

7. The amino acid sequence of the recombinant type V humanized collagen prepared in the present application is derived from a functional region of human natural type V collagen, and comprises the functional region and a similar functional region, as well as a protein with amino acid sequence mutated and modified respectively.

8. The application field of the recombinant type V humanized collagen prepared in the present application includes the preparation of high-end medical devices, such as biological dressings, human body bionic materials, plastic beauty materials, organoid culture, cardiovascular stent, coating, cardiac repair, tissue injection filling, ophthalmic materials, obstetrics and gynecology biomaterials, nerve repair and regeneration, liver tissue and vascular repair and regeneration, 3D printed artificial organ biomaterials, tumor prevention, bone repair materials, skin repair materials, kidney tissue repair, pancreas repair; high-end cosmetic raw materials and high-end pharmaceutical excipients; food additives and the like.

The advantages of the present application over the prior art include:

1. The present application provides the core functional region and amino acid sequence of recombinant type V humanized collagen.

2. The present application successfully biosynthesized recombinant type V humanized collagen for the first time.

3. The recombinant type V humanized collagen prepared in the present application has a good cell adhesion effect and does not cause an immune response when applied to the human body.

4. The preparation method is simple and the recombinant type V humanized collagen may be obtained on a large scale.

**Description of the Drawings**

Fig. 1: Electrophoretic detection diagram after crude purification of recombinant type V humanized collagen C5V3G1 and C5V3G2. The electropherogram after each purification step is shown.

Fig. 2: Electrophoresis detection diagram after crude purification of recombinant type V humanized collagen C5V3G3 and electrophoresis detection diagram after enzyme digestion of C5V3G1, C5V3G2, and C5V3G3. The electropherogram after each purification step is shown.

Fig. 3: Electrophoresis detection diagram after fine purification of recombinant type V humanized collagen C5V3G1, C5V3G2, and C5V3G3.

Fig. 4: Cell adhesion activity of recombinant type V humanized collagen C5V3G1.

Fig. 5: Cell adhesion activity of recombinant type V humanized collagen C5V3G2.

Fig. 6: Cell adhesion activity of recombinant type V humanized collagen C5V3G3.

**Detailed Description**

In order to make the purpose, technical solutions and advantages of the present application clearer, the technical solutions in the embodiments of the present application will be clearly and completely described below in combination with the embodiments of the present application. It is obvious that the described embodiments are a part of, but not all the embodiments of the present application. Based on the embodiments in the present application, all other embodiments obtained by those of ordinary skilled in the art without creative work fall within the scope of protection of the present application.

As used herein, a polypeptide refers to a plurality of amino acid residues linked by peptide bonds. Herein, a polypeptide comprises a plurality of repeating units derived from human type V collagen (GenBank: KAI4009058.1). A polypeptide may comprise a structure containing (repeating unit)$_n$ or (repeating unit)$_n$ -C-terminal region, and the repeating unit comprises either the amino acid sequence of SEQ ID NO. 1, or SEQ ID NO. 1 and an additional amino acid residue at the N-terminus and/or C-terminus of SEQ ID NO. 1. The number of the additional amino acid residue is 1-50. For example, the number of the additional amino acid residues is 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49. The additional amino acid residue may be derived from human type V collagen. For example, the additional amino acid residues may be consecutive amino acid residues to which the repeating unit is directly adjacent *in situ* in native human type V collagen. The additional amino acid residue may be the amino acid sequence set forth in SEQ ID NO. 7 or a consecutive amino acid segment thereof. Preferably, the additional amino acid residue may be a consecutive amino acid segment starting from position 1 of the amino acid sequence set forth in SEQ ID NO. 7. Herein, the additional amino acid residue may be located at the C-terminus.

Herein, each repeating unit may be directly linked or may be separated by one or more amino acid residues. The number n of the repeating unit may be 4-20, for example 5, 6, 7, 8, 9, 10, 11, 12, 13, 14, 15, 16, 17, 18 or 19. The polypeptide may comprise a C-terminal region. The C-terminal region may be a consecutive amino acid segment starting from position 1 of the repeating unit. The repeating unit may comprise the amino acid sequence of either SEQ ID NO. 2 or 3.

As used herein, the wording "segment" when used in conjunction with amino acids refers to a partial sequence that is smaller than a specified sequence. For example, the amino acid segment in SEQ ID NO. 7 refers to the partial sequence with any length less than the length of SEQ ID NO. 7. A "consecutive amino acids segment" refers to an amino acids segment composed of amino acids adjacent directly to each other in a specified sequence.

As used herein, with respect to a polypeptide or a particular amino acid sequence, "C-terminus" and "N-terminus" refer to the position opposite to the polypeptide or the particular amino acid sequence, specifically to the carboxyl or amino-terminal direction of the polypeptide or the particular amino acid sequence.

As used herein, when referring to a polypeptide or a particular amino acid sequence, the position is described relative to the C-terminus of the polypeptide or the particular amino acid sequence. For example, when referring to "from position 1 of the amino acid sequence of SEQ ID NO. 7 (galglk)", the amino acid at position 1 refers to G.

Herein, the repeating unit of the present application may comprise or may be the following sequence:

gkegtkgdpgpaglpgkdgppglrgfpgdrglpgpv (SEQ ID NO. 1);
gkegtkgdpgpaglpgkdgppglrgfpgdrglpgpvgal (SEQ ID NO. 2);
gkegtkgdpgpaglpgkdgppglrgfpgdrglpgpvgalglk (SEQ ID NO. 3);
gkegtkgdpgpaglpgkdgppglrgfpgdrglpgpvg (SEQ ID NO. 8);
gkegtkgdpgpaglpgkdgppglrgfpgdrglpgpvga (SEQ ID NO. 9);
gkegtkgdpgpaglpgkdgppglrgfpgdrglpgpvgalg (SEQ ID NO. 10); and
gkegtkgdpgpaglpgkdgppglrgfpgdrglpgpvgalgl (SEQ ID NO. 11).

As used herein, "nucleic acid" refers to a plurality of nucleotides linked by internucleotide linkage. The internucleotide linkage may be, for example, a phosphodiester linkage. The nucleic acid herein may comprise a polynucleotide encoding a polypeptide of the present application. In order to facilitate the subsequent processing of the polypeptide, the nucleic acid of the present application may also comprise nucleotides encoding a purification tag, such as a His tag, a GST tag, an MBP tag, a SUMO tag or a Nus tag, and nucleotides encoding a leader sequence when needed.

As used herein, the term "vector" is a nucleic acid carrier tool into which a polynucleotide is inserted. When a vector allows the expression of the protein encoded by the inserted polynucleotide, the vector is called an expression vector. The vector may be introduced into a host cell through either transformation, transduction or transfection, so that the genetic material elements it carries can be expressed in the host cell. The vector is well known to those skilled in the art, including but not limited to: plasmids; phagemids; cosmids; artificial chromosomes, for example, yeast artificial chromosomes (YAC), bacterial artificial chromosomes (BAC) or P1-derived artificial chromosomes (PAC); phages such as lambda phages or M13 phages; and animal viruses, and the like. The vector may contain a variety of elements for controlling expression, including but not limited to promoter sequences, transcription initiation sequences, enhancer sequences, selection elements, and reporter genes. In addition, the vector may also contain an origin of replication. The vector may comprise the nucleic acid of the present application to facilitate introduction into a cell for expression. The vector may comprise an expression control element, such as a promoter, a terminator, and/or an enhancer operably linked to said nucleic acid.

As used herein, the term "host cell" is a cell into which a nucleic acid molecule has been introduced by molecular biology techniques. These techniques include transfection with viral vectors, transformation with plasmid vectors, and introduction of naked DNA by electroporation, lipofection, and particle gun acceleration. The host cell may be a eukaryotic cell or a prokaryotic cell. The eukaryotic cell is, for example, a yeast cell, an animal cell and/or an insect cell. The prokaryotic cell may be an *Escherichia coli* cell.

Herein, each portion, for example the repeating unit or the C-terminal region, of the polypeptide of the present application may have certain mutations. For example, the amino acid sequence of one or more of these portions may have a substitution, deletion, addition or insertion of amino acid residue. That is, variants of each portion, for example repeating unit variants or C-terminal region variants, may be used in the present application, as long as the variants retain the activity in promoting cell adhesion. Specifically, a variant may have a certain percent identity, such as 80%, 85%, 90%, 91%, 92%, 93%, 94%, 95%, 96%, 97%, 98 %, and 99% identity to a specified sequence. The specified sequence may be any sequence of the present application, such as SEQ ID NOs. 1-6, but it is preferred that these variants retain the core sequence identified in the present application.

The polypeptides of the present application may be prepared by any suitable means, for example, by synthesis. Preferably, the polypeptide of the present application may be prepared by recombinant means. As a preparation method, the method may comprise any one or more of the following steps: (1) culturing the host cell according to claim 8 under a suitable culture condition; (2) harvesting the host cells and/or culture medium comprising the polypeptide; and (3) purifying the fusion protein or trimeric fusion protein. More specifically, the coding sequence of the polypeptide of the present application may be inserted into a suitable expression vector, such as PET-28a, to obtain a recombinant expression vector. The recombinant expression plasmid may be transformed into an *E. coli* competent cell BL21 (DE3), to obtain positive *E. coli* genetically engineered bacteria by screening. Then, the positive *E. coli* genetically engineered bacteria obtained by screening may be subjected to large-scale biological fermentation (for example, to be cultured in a constant temperature shaker at 220 rpm and 37°C for 7 hours). When cultured to a suitable cell density, cells may be induced to express proteins. For example, in the case of *E. coli* genetically engineered bacteria, (1) cool the shake flask after the culture to 16°C; (2) add IPTG stock solution for inducible expression; and (3) place the bacterial liquid after inducible expression into a centrifuge bottle, and collect the bacterial cells after centrifugation at 8000 rpm and 4°C for 10 min. The bacterial cells may be lysed (for example, through high-pressure homogenization) to obtain bacterial cell lysate, and then centrifuged to obtain a supernatant. The supernatant may be purified to obtain purified polypeptide. For example, the purification process may comprise one or more of the following steps: (1) crudely purifying the polypeptide on a Ni affinity chromatography column; (2) adding TEV enzyme at a certain ratio for enzyme digestion; and (3) finely purifying the polypeptide on an ion exchange column.

The polypeptide, nucleic acid, vector and/or host cell of the present application may be prepared into a composition or a kit. The composition or kit may comprise one or more of biological dressings, human body bionic materials, plastic beauty materials, organoid culture materials, cardiovascular stent materials, coating materials, tissue injection filling materials, ophthalmic materials, obstetrics and gynecology biomaterials, nerve repair and regeneration materials, liver tissue materials and vascular repair and regeneration materials, 3D printed artificial organ biomaterials, cosmetic raw materials, pharmaceutical excipients and food additives. The composition may be one or more of biological dressings, human body bionic materials, plastic beauty materials, organoid culture materials, cardiovascular stent materials, coating materials, tissue injection filling materials, ophthalmic materials, obstetrics and gynecology biomaterials, nerve repair and regeneration materials, liver tissue materials and vascular repair and regeneration materials, 3D printed artificial organ biomaterials, cosmetic raw materials, pharmaceutical excipients and food additives. The composition or kit may be used to promote cell adhesion *in vitro* or *in vivo*.

Examples

The following Examples are provided to illustrate the present application. Those skilled in the art should understand that the examples are merely illustrative but not limiting. The present application is limited only by the scope of the appended claims.

**Example 1: Construction and expression of recombinant type V humanized collagen**

1. Large-scale functional region screening was conducted to obtain the following different target gene segments of recombinant type V humanized collagen: (1) C5V3G1 amino acid sequence: gkegtkgdpgpaglpgkdgppglrgfpgdrglpgpvgalglk gkegtkgdpgpaglpgkdgppglrgfpgdrglpgpvgalglk gkegtkgdpgpaglpgkdgppglrgfpgdrglpgpvgalglk gkegtkgdpgpaglpgkdgppglrgfpgdrglpgpvgalglk gkegtkgdpgpaglpgkdgppglrgfpgdrglpgpvgalglk gkegtkgdpgpaglpgkdgppglrgfpgdrglpgpvgalglk (C5V3G1 amino acid sequence: SEQ ID NO. 6; with repeating sequence SEQ ID NO.3, gkegtkgdpgpaglpgkdgppglrgfpgdrglpgpvgalglk); (2) C5V3G2 amino acid sequence: gkegtkgdpgpaglpgkdgppglrgfpgdrglpgpvgal gkegtkgdpgpaglpgkdgppglrgfpgdrglpgpvgal gkegtkgdpgpaglpgkdgppglrgfpgdrglpgpvgal gkegtkgdpgpaglpgkdgppglrgfpgdrglpgpvgal gkegtkgdpgpaglpgkdgppglrgfpgdrglpgpvgal gkegtkgdpgpaglpgkdgppglrgfpgdrglpgpvgal gkegtkgdpgpaglpgkdgppglrgfpgdrglpgpvgal (C5V3G2 amino acid sequence SEQ ID NO: 5, with repeating unit SEQ ID NO. 2, gkegtkgdpgpaglpgkdgppglrgfpgdrglpgpvgal); and (3) C5V3G3 amino acid sequence: gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv gkegtkgdpgpaglpgkdgppglrgfpgdrglpgpv (C5V3G3 amino acid sequence: SEQ ID NO: 4, with the repeating unit of SEQ ID NO. 1: gkegtkgdpgpaglpgkdgppglrgfpgdrglpgpv).

The synthesized gene segments (C5V3G1 base sequence:

GGGAAAGAAGGAACGAAAGGTGACCCTGGCCCAGCGGGTCTGCC

GGGCAAGGACGGTCCACCGGGTCTGCGCGGTTTCCCGGGCGACCGCG

GCCTGCCGGGTCCGGTTGGCGCGTTGGGTCTTAAGGGCAAAGAAGGC

ACCAAAGGAGATCCGGGTCCGGCTGGTTTACCGGGCAAGGATGGTCC

GCCGGGTCTGCGTGGTTTTCCGGGTGACCGTGGTCTGCCGGGTCCGGT

CGGAGCCCTGGGTTTGAAGGGCAAAGAGGGCACCAAAGGCGACCCA

GGACCGGCAGGCCTCCCGGGTAAGGATGGCCCTCCGGGGCTGCGTGG

TTTTCCGGGTGATCGCGGCTTGCCGGGACCGGTTGGTGCGCTGGGTCT

TAAGGGTAAAGAAGGCACCAAAGGTGACCCGGGCCCGGCGGGTCTGC

CGGGCAAAGATGGTCCGCCAGGCCTGAGAGGCTTCCCGGGTGACCGC
GGCTTGCCGGGTCCGGTTGGTGCGCTGGGCCTTAAGGGTAAAGAGGG
CACCAAAGGCGATCCGGGTCCGGCTGGCCTGCCGGGGAAGGACGGCC
CACCGGGCCTGCGTGGTTTTCCGGGCGACCGTGGTTTGCCGGGCCCGG
TGGGCGCTCTGGGTCTGAAAGGGAAAGAGGGTACGAAGGGTGATCCG
GGTCCGGCAGGCTTGCCGGGCAAGGACGGCCCGCCTGGCCTGCGTGG
TTTCCCGGGTGACCGTGGTTTACCGGGTCCCGTGGGCGCGCTGGGTCT
GAAAGGTAAGGAAGGCACCAAGGGTGATCCGGGCCCGGCCGGTCTGC
CGGGCAAGGACGGTCCGCCCGGATTACGTGGCTTCCCGGGTGATCGTG
GTCTGCCGGGTCCTGTAGGTGCGCTGGGTCTCAAGGGCAAAGAGGGT
ACTAAGGGTGATCCGGGCCCGGCGGGTCTGCCAGGCAAAGACGGCCC
ACCGGGCTTGCGCGGTTTTCCGGGCGATCGTGGTTTGCCGGGTCCTGT
GGGCGCACTGGGCTTGAAGTAA, SEQ ID NO:12; C5V3G2 base sequence:
GGGAAAGAAGGAACCAAGGGTGACCCGGGGCCAGCCGGTTTGCCGG
GTAAGGATGGTCCGCCTGGCTTGCGTGGTTTTCCGGGGGACCGTGGTT
TGCCGGGTCCGGTTGGTGCGCTGGGTAAAGAAGGCACGAAGGGCGAC
CCGGGCCCAGCCGGTTTACCGGGGAAAGATGGTCCTCCGGGCCTGCGC
GGTTTTCCGGGAGACCGTGGTCTTCCGGGCCCGGTGGGTGCGCTGGGC
AAGGAGGGCACCAAAGGTGACCCTGGCCCTGCGGGTCTGCCGGGTAA
GGACGGTCCACCGGGTCTGCGTGGCTTTCCGGGCGACCGTGGCTTGCC
CGGTCCGGTTGGCGCGCTGGGTAAAGAGGGTACGAAGGGTGACCCGG
GTCCTGCGGGCCTGCCGGGCAAGGACGGCCCGCCGGGACTGCGCGGA
TTCCCGGGCGATCGCGGCCTCCCGGGTCCGGTCGGTGCGCTGGGCAAA
GAAGGTACTAAAGGTGATCCGGGCCCGGCGGGCCTGCCGGGCAAGGA
TGGCCCGCCGGGTTTGCGCGGTTTCCCGGGCGACCGTGGTTTGCCGGG
CCCAGTGGGCGCACTGGGTAAGGAGGGCACCAAAGGCGATCCGGGAC
CAGCTGGTCTGCCGGGCAAGGACGGCCCACCGGGTCTCAGAGGCTTC
CCGGGGGACCGCGGTCTGCCGGGCCCGGTAGGCGCACTGGGTAAAGA
AGGCACCAAAGGTGACCCGGGTCCGGCTGGTCTGCCGGGTAAGGATG
GCCCGCCGGGCCTGCGTGGCTTCCCGGGTGATCGTGGCTTGCCTGGCC
CAGTGGGTGCACTGGGCAAAGAGGGCACCAAGGGTGATCCGGGTCCG

GCGGGTCTGCCGGGTAAAGATGGTCCACCGGGTTTACGTGGTTTTCCG GGCGATCGTGGTCTCCCGGGCCCGGTTGGCGCTCTGTAA, SEQ ID NO:13;

and C5V3G3 base sequence: GGGAAAGAAGGAACGAAAGGCGATCCGGGTCCGGCGGGCCTGCCGG GTAAGGACGGCCCGCCGGGATTACGCGGTTTCCCGGGTGACCGCGGTT TGCCGGGTCCGGTTGGCAAA-GAAGGTACTAAGGGTGATCCGGGCCCA GCCGGTCTGCCGGGCAAGGATGGTCCGCCAGGCCTGA-GAGGCTTCCC GGGTGACCGCGGGCTGCCGGGCCCGGTTGGTAAGGAGGGCACCAAAG GCGATCCGGGTCCGGCGGGTTTGCCGGGCAAGGATGGCCCGCCAGGC CTGCGTGGCTTTCCGGGTGATCGTGGTCTGCCGGGCCCCGTGGGCAAA GAGGGCACCAAAGGT-GATCCGGGTCCAGCGGGTCTGCCAGGTAAGGA CGGTCCGCCTGGCCTGCGTGGTTTCCCGGGTGATCGTGGTCTGCCGGG TCCGGTCGGTAAAGAAGGCAC-CAAGGGCGACCCGGGCCCGGCAGGGC TGCCGGGTAAGGATGGCC-CACCGGGTCTGCGTGGTTTTCCGGGCGACC GTGGTCTTCCGGGTCCGGTCGGTAAAGAGGGCAC-CAAAGGTGACCCG GGTCCCGCGGGTCTGCCGGGTAAGGACGGCCCTCCGGGCCTGCGCGG CTTCCCGGGCGATCGCGGTCTGCCGGGTCCGGTTGGTAAAGAAGGTAC GAAGGGCGATCCTGGTCCAG-CAGGCTTGCCGGGCAAAGATGGTCCGC CCGGCCTTCGCGGATTCCCGGGC-GACCGTGGCCTGCCGGGTCCGGTGG GTAAAGAAGGCACCAAGGGC-GACCCGGGTCCTGCTGGCTTGCCGGGC AAGGACGGCCCACCGGGTTTACGCGGTTTTCCGGGC-GATCGTGGTTTG CCGGGTCCGGTGGGCAAAGAGGGAACCAAAGGTGACCCTGGCCCAGC TGGCTTGCCGGGCAAAGATGGTCCGCCGGGTCTGCGTGGCTTTCCGGG TGACCGTGGTTTGCCGGGCCCGGTTGGTAAGGAGGGCACCAAAGGCG AC-CCGGGTCCGGCGGGTCTGCCGGGTAAGGACGGTCCACCGGGCTTA CGTGGTTTCCCGGGGGACCGTGGCCTGCCGGGCCCCGTGTAA, SEQ ID NO:14) were inserted between the Kpn I and Xho I restriction sites of the pET-32a expression vector to obtain a recombinant expression plasmid.

2. The successfully constructed expression plasmid was transformed into *E. coli* competent cells BL21(DE3). The specific processes were as follows. (1) The *E. coli* competent cells BL21(DE3) in an ultra-low temperature refrigerator were taken out and placed on ice; 2 μl of the plasmid to be transformed was added to the *E. coli* competent cells BL21(DE3) when half melted, and mixed slightly for 2-3 times. (2) The mixture was placed on ice for an ice bath of 30 min, then heat shocked by a water bath at 42°C for 45-90 seconds, and taken out followed by placed on ice for an ice bath of 2 min. (3) The mixture was transferred to a biosafety cabinet and added with 700μl LB liquid culture medium, then cultured at 37°C, 220 rpm for 60min. (4) 200μl of bacterial liquid was taken and evenly spread on an LB plate containing ampicillin sodium. (5) The plate was cultured in an incubator at 37°C for 15-17 h until colonies of uniform size grow.

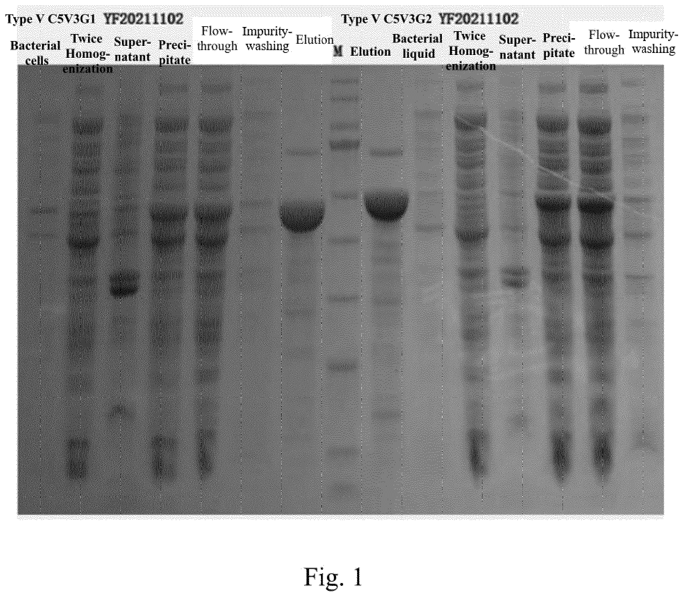

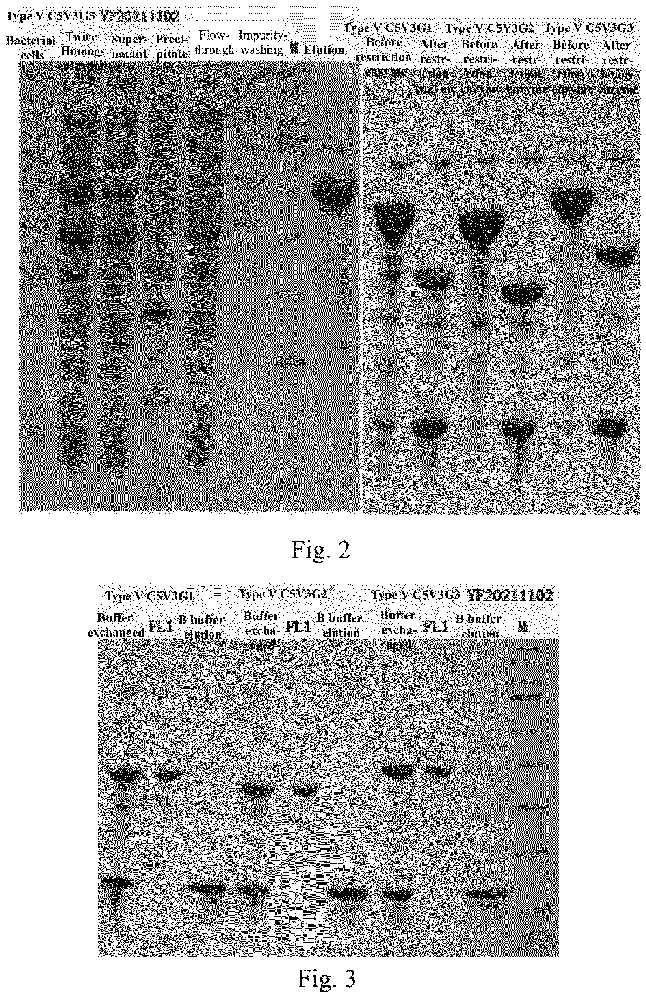

3. 5-6 single colonies were picked from the transformed LB plate to shake flasks containing antibiotic stock solution, and placed in a constant temperature shaker at 220 rpm and 37°C for 7h. Then the cultured shake flasks were cooled to 16°C, and added with IPTG to induce expression for a period of time. Then the bacterial liquid was divided into centrifuge bottles and centrifuged at 8000 rpm and 4°C for 10 min. Then the bacterial cells were collected and the weight of the bacterial cells was recorded. Samples were taken for electrophoresis detection. (See the lane "Bacterial cells" in Fig. 1 and Fig. 2).

4. The collected bacterial cells were resuspended in a balanced working buffer, and the bacterial liquid was cooled to ≤15°C, and homogenized through high-pressure homogenization twice. After completion, the bacterial liquid was collected. The homogenized bacterial liquid was dispensed into centrifuge bottles, and centrifuged at 17000 rpm and 4°C for 30 min. The supernatant was collected, and the supernatant and precipitate were taken for electrophoresis detection. (See Fig. 1 and Fig. 2, in which "Twice homogenization" represents the electropherogram of the bacterial liquid after high-pressure homogenization twice, and "Supernatant" and "Precipitate" represent the electropherogram of the supernatant and precipitate, respectively).

5. The recombinant humanized type V collagen was purified and enzymatically digested. The specific processes were as follows. (1) Crude purification: a. washing the column material with water; b. column material equilibration; c. sample loading: adding the centrifuged supernatant to the column until the liquid drained completely, and the flow-through was taken for electrophoresis test (see lane "Flow-through" in Fig. 1 and Fig. 2); d. washing of impure proteins: adding 25 mL of washing buffer (200 mM sodium chloride, 25 mM Tris, 20 mM imidazole) until the liquid drained completely, and the washing flow-through was taken for electrophoresis testing (see lane "Impurity-washing" in Fig. 1 and Fig. 2); e. collection of the target protein: adding 20 mL of elution buffer and collecting the flow-through to obtain the target protein containing His tag, detecting the protein concentration using UV-visible spectrophotometry with a calculation of the protein concentration according to the following formula (C (mg/ml) = A280× dilution fold × extinction coefficient); and conducting an electrophoresis detection (see lane "Elution" in Fig. 1 and Fig. 2); f. washing the column with 1M imidazole working buffer; and g. washing the column material with purified water. (2) Enzyme digestion: adding TEV enzyme at a 20:1 ratio of the total amount of proteins to the total amount of TEV enzymes for enzyme digestion at 16°C for 2 hours, and sampling for electrophoresis detection. The solution of the enzymatically digested protein was put into a dialysis bag, dialyzed at 4°C for 2 hours, and then transferred into new dialysate for dialysis at 4°C overnight. (See Fig. 3 for the electropherogram after changing the dialysate). (3) Fine purification: a. column material equilibration: equilibrating the column material by using A buffer (20 mM Tris, 20 mM sodium chloride) with a flow rate of 10 ml/min; b. sample loading: loading the sample and collecting the flow-through (see Fig. 3, labeled FL1) with a flow rate of 5 ml/min, obtaining recombinant type V humanized collagen C5V3G1, C5V3G2 and C5V3G3, and performing electrophoresis detection, as shown in Fig. 3; c. elution: eluting 3 CVs with a set of 100% B buffer (20mM Tris, 1M sodium chloride, pH8.0), collecting the peaks, and performing electrophoresis detection (for the B buffer elution buffer, see lane "B buffer elution" in Fig. 3); d. washing the column material; e. storing the protein in a 4°C environment. Fig. 3 shows that the apparent molecular weights of recombinant type V humanized collagen C5V3G1, C5V3G2 and C5V3G3 are 32kDa, 29kDa and 34kDa, respectively, which are consistent with the predicted molecular weights.

**Example 2: Detection of biological activity of recombinant type V humanized collagen**

The method for detecting the activity of collagen may refer to Juming Yao, Satoshi Yanagisawa, Tetsuo Asakura, Design, Expression and Characterization of Collagen-Like Proteins Based on the Cell Adhesive and Crosslinking Sequences Derived from Native Collagens, J Biochem. 136, 643-649(2004). The specific implementation methods are as follows.

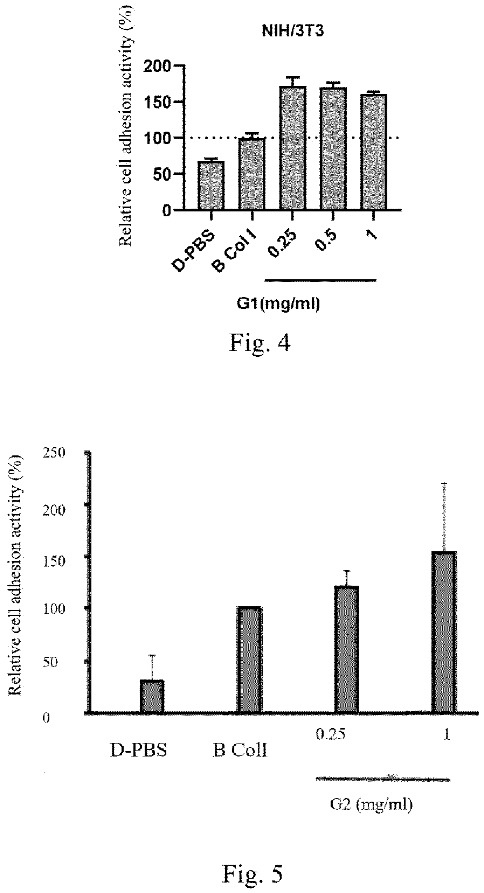

(1) Ultraviolet absorption method was used to detect the concentration of protein samples to be tested, including bovine type I collagen (China Institute of Food and Drug Control, No.: 380002; 0.5mg/mL, positive well), and recombinant type V humanized collagen proteins C5V3G1, C5V3G2, C5V3G3 provided by the present application. Specifically, the ultraviolet light absorption of the sample was assayed at 215 nm and 225 nm respectively, and the protein concentration was calculated using the empirical formula C ($\mu$g/mL) = 144 $\times$ (A215-A225). Note that the detection should be performed in the case of A215<1.5. The principle of the method is: to measure the characteristic absorption of peptide bonds at far-ultraviolet light, which is not affected by the chromophore content, with few interfering substances, The method is easy to operate, and is suitable for the detection of human collagen and analogs thereof that do not develop color in Coomassie brilliant blue. (Reference is Walker JM. The Protein Protocols Handbook, second edition. HumanaPress. 43-45.). After the detection of the protein concentration, the concentration of all proteins to be tested was adjusted to 0.25mg/mL, 0.5mg/mL or 1mg/ml with PBS.
(2) 100 $\mu$L of various protein solutions and blank PBS solution control (see the D-PBS groups in Fig. 4 - Fig. 6) were added to the 96-well plate, and let stand at room temperature for 60 min.
(3) $10^5$ 3T3 cells in good culture status were added into each well, and incubated at 37°C for 60 min.
(4) Each well was washed 4 times with PBS.
(5) The absorbance at OD492nm was detected with an LDH detection kit (Roche, 04744926001). The cell adhesion rate can be calculated according to the numerical value of the blank control. The calculation formula was as follows:

$$\text{cell adhesion rate} = \frac{\text{test well} - \text{blank well}}{\text{postive well} - \text{blank well}} \times 100\%.$$

The adhesion rate of cells (relative cell adhesion activity) can reflect the activity of collagen. The higher the activity of the protein, the better it can provide cells with a high-quality external environment in a short time to help cell adhesion.

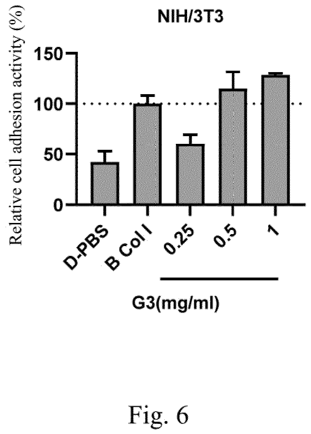

The results are shown in Figs. 4, 5, and 6. By comparison, it can be seen that the recombinant type V humanized collagen of the present application has better bioadhesion activity compared with bovine type I collagen (B ColI group, 0.5mg/ml). Fig. 4 depicts the relative cell adhesion activity of C5V3G1 relative to bovine type I collagen, indicating that CSV3G1 has cell adhesion activity and has higher cell adhesion activity than bovine type I collagen at concentrations of 0.25, 0.5 and 1mg/ml. Fig. 5 shows the relative cell adhesion activity of C5V3G2 relative to bovine type I collagen, indicating that C5V3G2 has better cell adhesion activity at concentrations above 0.25 mg/ml. Fig. 6 shows the relative cell adhesion activity of C5V3G3 relative to bovine type I collagen, indicating that C5V3G3 has cell adhesion activity and has higher cell adhesion activity than bovine type I collagen at a concentration of 0.5mg/ml.

**Example 3: Mass spectrometry detection of recombinant type V humanized collagen**

Experimental method

| Equipment name | Matrix-assisted laser desorption ionization-time of flight mass spectrometer, MALDI-TOF/TOF Ultraflextreme™, Brucker, Germany | | |
|---|---|---|---|
| Matrix | CHCA | Laser Energy | 125 |
| Data retrieval Software | Mascot | Retrieved Species | ALL entries |
| Search database | NCBIprot | | |

After the reduction with DTT and alkylation with iodoacetamide, the protein sample was enzymatically digested overnight with trypsin. The peptide segments obtained after enzyme digestion were then desalted by C18ZipTip, mixed with the matrix α-cyano-4-hydroxycinnamic acid (CHCA), and spotted on plates. Finally, analysis was performed using the matrix-assisted laser desorption ionization-time of flight mass spectrometer MALDI-TOF/TOF Ulraflextreme™, Brucker, Germany (the technology of peptide fingerprinting referred to Protein J.2016;35:212-7). Data retrieval was handled via the MS/MS Ion Search page on the local masco website. The protein identification results were obtained based on the primary mass spectrometry of the peptide segments resulting from enzyme digestion. Parameter: Trypsin digestion was detected and two missed cleavage sites were set. The alkylation of cysteine was set as a fixed modification. The oxidation of methionine was set as a variable modification. The database used for identification was NCBprot.

Table 1: Molecular weight detected by mass spectrometry and corresponding peptides for C5V3G1

| Observed value | Mr (predicted value) | Peptide |
|---|---|---|
| 1565.7522 | 1564.7450 | GKEGTKGDPGPAGLPGK |
| 2073.0474 | 2072.0401 | EGTKGDPGPAGLPGKDGPPGLR |
| 1657.8545 | 1656.8472 | GDPGPAGLPGKDGPPGLR |
| 711.3766 | 710.3693 | DGPPGLR |
| 1707.8803 | 1706.8730 | GFPGDRGLPGPVGALGLK |
| 1078.6592 | 1077.6520 | GLPGPVGALGLK |

The coverage rate of the detected polypeptide segments was 100%, which was consistent with the theoretical sequence, thus the detection results were very credible.

Table 2: Molecular weight detected by mass spectrometry and corresponding peptides for C5V3G2

| Observed value | Mr (predicted value) | Peptide |
|---|---|---|
| 1565.7522 | 1564.7450 | EGTKGDPGPAGLPGKDGPPGLR |
| 711.3766 | 710.3693 | GDPGPAGLPGKDGPPGLR |
| 1707.8803 | 1706.8730 | DGPPGLR |
| 1078.6592 | 1077.6520 | GLPGPVGALGK |

The coverage rate of the detected polypeptide segments was 83% compared with the theoretical sequence, thus the detection results were very credible.

Table 3: Molecular weight detected by mass spectrometry and corresponding peptides for C5V3G3

| Observed value | Mr (predicted value) | Peptide |
|---|---|---|
| 711.3914 | 710.3842 | DGPPGLR |
| 1353.7538 | 1352.7465 | GFPGDRGLPGPVGK |

(continued)

| Observed value | Mr (predicted value) | Peptide |
|---|---|---|
| 1657.8985 | 1656.8912 | GDPGPAGLPGKDGPPGLR |
| 2073.1050 | 2072.0977 | EGTKGDPGPAGLPGKDGPPGLR |

The coverage rate of the detected polypeptide segments was 96.11% compared with the theoretical sequence, thus the detection results were very credible.

The above examples are preferred embodiments of the present application, but the embodiments of the present application are not limited by the above examples. Any other changes, modifications, substitutions, combinations, or simplifications without departing from the spirit and principles of the present application shall be equivalent replacements and shall be encompassed in the scope of protection of the present application.

**Claims**

1. A polypeptide comprising a structure of (repeating unit)$_n$ or (repeating unit)$_n$ -C-terminal region, wherein the repeating unit comprises either the amino acid sequence of SEQ ID NO. 1, or SEQ ID NO. 1 and an additional amino acid residue at the N-terminus and/or C-terminus of SEQ ID NO. 1, and the number of the additional amino acid residue is 1-50; each repeating unit is directly linked and the number n of the repeating unit is 4-20, and the C-terminal region is a consecutive amino acid segment starting from position 1 of the repeating unit; preferably the polypeptide is recombinant type V humanized collagen.

2. The polypeptide according to claim 1, wherein the additional amino acid residue is at the C-terminus of SEQ ID NO. 1 and is the amino acid sequence set forth in SEQ ID NO. 7 or a consecutive amino acid segment thereof.

3. The polypeptide according to claim 2, wherein the additional amino acid residue is a consecutive amino acid segment starting from position 1 of the amino acid sequence of SEQ ID NO. 7.

4. The polypeptide according to any one of claims 1-3, wherein the repeating unit comprises the amino acid sequence of any one of SEQ ID NOs. 2, 3 and 8-11.

5. The polypeptide according to any one of claims 1-4 comprising an amino acid sequence selected from SEQ ID NOs. 4-6.

6. A nucleic acid comprising nucleotides encoding the polypeptide of any one of claims 1 to 5, wherein, optionally, the nucleic acid further comprises nucleotides encoding a purification tag, e.g. a His tag, a GST tag, an MBP tag, a SUMO tag or a NusA tag; optionally, the nucleic acid further comprises nucleotides encoding a leader sequence.

7. A vector comprising the nucleic acid according to claim 6, optionally comprising an expression control element, such as a promoter, a terminator and/or an enhancer, operably linked to the nucleic acid.

8. A host cell comprising the nucleic acid according to claim 6 or the vector according to claim 7; wherein preferably, the host cell is a eukaryotic cell or a prokaryotic cell; wherein preferably, the eukaryotic cell is a yeast cell, an animal cell and/or an insect cell, and/or the prokaryotic cell is an *Escherichia coli* cell, for example *E. coli* BL21.

9. A method for producing of the polypeptide according to any one of claims 1-5, comprising:

   (1) culturing the host cell according to claim 8 under a suitable culture condition;
   (2) harvesting the host cell and/or culture medium comprising the polypeptide; and
   (3) purifying fusion protein, by for example (1) purification on a Ni affinity chromatography column to obtain a crude polypeptide; (2) adding TEV enzyme at a certain ratio for enzyme digestion; (3) purification on an ion exchange column to obtain a refined polypeptide.

10. A composition comprising the polypeptide according to any one of claims 1-5, the nucleic acid according to claim 6, the vector according to claim 7 and/or the host cell according to claim 8.

11. The composition according to claim 10, which is one or more of biological dressings, human body bionic materials, plastic beauty materials, organoid culture materials, cardiovascular stent materials, coating materials, tissue injection filling materials, ophthalmic materials, obstetrics and gynecology biomaterials, nerve repair and regeneration materials, liver tissue materials and vascular repair and regeneration materials, 3D printed artificial organ biomaterials, cosmetic raw materials, pharmaceutical excipients and food additives.

12. Use of the polypeptide according to any one of claims 1-5, the nucleic acid according to claim 6, the vector according to claim 7, the host cell according to claim 8 and/or the composition according to claim 10 for use in promoting cell adhesion *in vitro* or in the manufacture of a product or a kit for promoting cell adhesion.

13. Use of the polypeptide according to any one of claims 1-5, the nucleic acid according to claim 6, the vector according to claim 7, the host cell according to claim 8 and/or the composition according to claims 10 for the manufacture of high-end medical devices, such as biological dressings, human body bionic materials, plastic beauty materials, organoid culture, cardiovascular stent, coating, tissue injection filling, ophthalmic materials, obstetrics and gynecology biomaterials, nerve repair and regeneration, liver tissue and vascular repair and regeneration, 3D printed artificial organ biomaterials; high-end cosmetic raw materials and high-end pharmaceutical excipients; and food additives.

Fig. 1

Fig. 2

Fig. 3

Fig. 4

Fig. 5

Fig. 6

## INTERNATIONAL SEARCH REPORT

| International application No. |
|---|
| **PCT/CN2023/087212** |

### A. CLASSIFICATION OF SUBJECT MATTER

C07K14/78(2006.01)i；C07K1/18(2006.01)i；C12N15/12(2006.01)i；C12N1/21(2006.01)i；C12N15/63(2006.01)i；C12N15/70(2006.01)i；A61K8/65(2006.01)i；A61K38/39(2006.01)i；A61L31/04(2006.01)i；A61K47/42(2017.01)i；A61L27/24(2006.01)i；A23L33/17(2016.01)i；A61L15/32(2006.01)i；A61Q19/00(2006.01)i；C07K19/00(2006.01)i

According to International Patent Classification (IPC) or to both national classification and IPC

### B. FIELDS SEARCHED

Minimum documentation searched (classification system followed by classification symbols)

IPC：C07K,C12N,A61K,A61L,A23L,A61Q

Documentation searched other than minimum documentation to the extent that such documents are included in the fields searched

Electronic data base consulted during the international search (name of data base and, where practicable, search terms used)

CNABS; CNMED; CNTXT; VEN; DWPI; SIPOABS; EPTXT; WOTXT; USTXT; JPTXT; CNKI; 中国专利生物序列检索系统, Chinese Patent Biological Sequence Retrieval System; Genbank; NCBI; EMBL; STN; Elsevier; WEB OF SCIENCE; Pubmed: 胶原蛋白, V型, 重组, 重复单元, collagen, type V, recombinant collagen, SEQ ID Nos.: 1-6, 8-11等

### C. DOCUMENTS CONSIDERED TO BE RELEVANT

| Category* | Citation of document, with indication, where appropriate, of the relevant passages | Relevant to claim No. |
|---|---|---|
| A | CN 112745385 A (JIANGSU JINGSEN BIOLOGICAL MEDICINE NEW MATERIAL TECHNOLOGY CO., LTD.) 04 May 2021 (2021-05-04) see abstract, claims 1-8, SEQ ID NO: 1, and description, paragraphs 18-21 | 1-13 |
| A | CN 113476593 A (SHANXI JINBO BIO-PHARMACEUTICAL CO., LTD.) 08 October 2021 (2021-10-08) see abstract, and claims 1-10 | 1-13 |
| A | US 2021002349 A1 (UNIVERSITY OF TSUKUBA) 07 January 2021 (2021-01-07) see abstract, and claims 1-24 | 1-13 |
| A | 李阳 (LI, Yang). "重组胶原蛋白的绿色生物制造及其应用 (Green Biological Manufacture and Application of Recombinant Collagen)" 化工进展 (Chemical Industry and Engineering Progress), Vol. 40, No. 3, 13 January 2021 (2021-01-13), see abstract, pages 1263-1271 | 1-13 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 July 2023** | **02 August 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 512 824 A1**

| | |
|---|---|
| **INTERNATIONAL SEARCH REPORT** | International application No.<br>**PCT/CN2023/087212** |

**C.    DOCUMENTS CONSIDERED TO BE RELEVANT**

| Category* | Citation of document, with indication, where appropriate, of the relevant passages | Relevant to claim No. |
|---|---|---|
| A | CN 114316030 A (XI'AN GIANT BIOGENE TECHNOLOGY CO., LTD.) 12 April 2022 (2022-04-12)<br>    see abstract, and claims 1-6 | 1-13 |
| A | M Hatai. "Effects of fibronectin-type V collagen recombinant fusion protein on cell adhesion and cell proliferation"<br>*Cell Struct Funct.*, Vol. 17, No. 5, 31 October 1992 (1992-10-31),<br>    see abstract, and pages 294-299 | 1-13 |

Form PCT/ISA/210 (second sheet) (July 2022)

21

**INTERNATIONAL SEARCH REPORT**

International application No.

**PCT/CN2023/087212**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2023/087212**

| Patent document cited in search report | | | Publication date (day/month/year) | Patent family member(s) | | | Publication date (day/month/year) |
|---|---|---|---|---|---|---|---|
| CN | 112745385 | A | 04 May 2021 | None | | | |
| CN | 113476593 | A | 08 October 2021 | None | | | |
| US | 2021002349 | A1 | 07 January 2021 | WO | 2019098246 | A1 | 23 May 2019 |
| CN | 114316030 | A | 12 April 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210849498

**Non-patent literature cited in the description**

- **JUMING YAO** ; **SATOSHI YANAGISAWA** ; **TETSUO ASAKURA**. Design, Expression and Characterization of Collagen-Like Proteins Based on the Cell Adhesive and Crosslinking Sequences Derived from Native Collagens. *J Biochem.*, 2004, vol. 136, 643-649

- **WALKER JM**. The Protein Protocols Handbook. HumanaPress, 43-45
- *Protein J.*, 2016, vol. 35, 212-7